# EUROPEAN PATENT SPECIFICATION

(11) **EP 1 994 972 B1**
(45) Date of publication and mention of the grant of the patent: **06.07.2011**
(21) Application number: 07108268.9
(22) Date of filing: 15.05.2007
(51) Int. Cl.: B01D 15/18, G01N 30/42, C07K 1/16, A23J 1/14

(54) **Method and device for continuous chromatographic separations**
Verfahren und Vorrichtung für kontinuierliche chromatografische Trennung
Procédé et dispositif pour séparations chromatographiques en continu

(43) Date of publication of application: 26.11.2008
(73) Proprietor: Coöperatie AVEBE U.A., 9641 GK Veendam (NL)
(72) Inventor: Bisschops, Marc Antonius Theodorus, 4813 CS Breda (NL); Giuseppin, Marco Luigi Federico, 9461 JB Gieten (NL)
(74) Representative: van Loon, C.J.J.

(56) References cited:
- WO-A-99/65586
- WO-A-2004/082397
- US-A- 4 593 477
- US-A- 5 223 143
- S. GHOSE, H. A. CHASE , N. TITCHENER-HOOKER: "Bed height monitoring and control for expanded bed chromatography" BIOPROCESS AND BIOSYSTEMS ENGINEERING, vol. 23, no. 6, December 2000 (2000-12), pages 701-708, XP002454342
- TRAVIS V. THELEN, W. FRED RAMIREZ: "Monitoring, modeling, and control strategies for expanded-bed adsorption processes" BIOSEPARATION, vol. 8, no. 1-5, January 1999 (1999-01), pages 11-31, XP002454343

## Description

### FIELD OF THE INVENTION

The invention relates to a method and device for chromatographic separations. In particular, the invention relates to an expandable bed column system that is suitable for obtaining agro en dairy isolates, for instance, a native potato protein isolate.

### BACKGROUND OF THE INVENTION

The potato belongs to the Solanaceae or nightshade family whose other members include tomatoes, eggplants, peppers, and tomatillos.

In the professional vocabulary the undiluted juice from the potato tuber is called potato fruit juice, whereas the diluted juice is designated potato fruit water. Potato fruit juice may be produced by washing and rasping potatoes and separating the starch and fibres by various techniques, such as centrisieves, hydrocyclones and decanters.

Processed, deproteinized and concentrated potato fruit water is used as feed supplement by evaporation or reverse osmosis is used by some potato starch manufacturers. Reverse osmosis, which is not as energy demanding as evaporation, does however demand that the potato fruit water is pre-treated and filtered clear to avoid clogging of the membranes which hold inorganic salts and low molecular organic components back in the concentrate.

Fresh potato juice is a complex mixture of soluble and insoluble material comprising proteins, starch, minerals, toxic glycoalkaloids, and monomeric and polymeric reactive phenols. Fresh potato fruit juice is however not stable. The oxidation of natural phenolic compounds in potato juice causes them to turn brown or black. Chemically, the phenolic compounds are oxidized into quinones, which rapidly combine into a dark polymer residue. During the oxidation process reaction and partial crosslinking of the proteins may occur very rapidly. This crosslinking and other chemical modifications dramatically reduce the solubility of proteins and sediments occur.

Thus, from a technological point of view the complexity and instability of the potato juice makes the separation and isolation of minimally denatured or modified potato proteins much more complicated and economically demanding than the isolation of proteins from other types of protein solution such as ewe milk.

Native potato proteins can tentatively be divided into three classes (i) the patatin family, highly homologous acidic 43 kDa glycoproteins (40-50% of the potato proteins), (ii) basic 5-25 kDa protease inhibitors (30-40% of the potato proteins) and (iii) other proteins mostly high molecular weight proteins (10-20% of the potato proteins) (Pots et. al., J. Sci. Food Agric 79 (1999) 1557-1564.

Patatin is a family of glycoproteins that have lipid acyl hydrolase and transferase activities and account for up to 40% of the total soluble protein in potato tubers.

However, its nutritional qualities (i.e. protein efficiency ratio and biological value) have been shown to be greater than that of casein and comparable to that of whole egg. Potato protein is rich in lysine and theoretically an excellent supplement for lysine-poor proteins such as those of cereals. Despite its unique nutritional qualities, potato protein is currently only used as animal feed, because the available products exhibit a number of serious drawbacks.

One of the major drawbacks is that the recovery of potato protein from the effluent of potato starch mills is commonly carried out on an industrial scale by heat coagulation and /or acid precipitation.

Prior attempts to isolate the proteins from the potato juice by more mild methods, such as membrane filtration and precipitation have proven to be inefficient in the industrial scale production environment. Membrane filtration applied directly to unclarified and clarified potato juice has proven to be very complicated and inefficient due to heavy fouling of the membranes and concomitant loss of flux and separation ability. Both membrane filtration and precipitation methods have significant drawbacks when applied directly to the potato juice due to the lack of selectivity between the desired protein product and other components in the raw material. Membrane filtration, for example, cannot separate the high molecular weight protein product from polymerized phenolic compounds or polysaccharides since the membrane will tend to retain them all. Furthermore, it may lead to inferior protein quality in terms of flavour and functionality in foods.

Due to the presently applied heat coagulation processes, potato protein becomes heavily denatured and as a consequence becomes devoid of functional properties, i.e. emulsifying capacity, foaming capacity, thermogelling capacity, water binding capacity and biological activities. However this variant is poorly soluble in water, reducing its functionality and rendering it less attractive for the food industry.

There is a commercial interest to produce native potato protein fractions. Here native potato protein is defined as the protein without any significant physical or (bio)chemical modification or inactivation. WO2004/082397 suggest the use of an expandable bed adsorption method of a continuous mode type for the isolation of biomolecules. However, for continuous systems, the application of expandable bed adsorption columns poses a challenge in view of the expandable nature of the adsorption which may affect the throughput between various expandable bed columns.

S. Chose, H.A Chase, N. Titchener-Hooker: "Bed height monitoring and control for expanded bed chromatography" Bioprocess and biosystems engineering, vol. 23, no. 6, December 2000 (2000-12), pages 701 - 708, shows an expanded bed level control for a single column. A LED sensor and pump algorithm is disclosed. The publication is silent for multiple column systems.
WO99/65586 shows an expanded bed column system. The publication does not discuss multiple column arrangments. Headspace control is discussed, by means of a sensor that detects a distance between the fluid level and the expanded level, so that a correct expansion is obtained and the amount of bufferfluid can be limited.

### SUMMARY OF THE INVENTION

According to one aspect of the invention, a chromatographic separation method is provided comprising: providing a plurality of expandable bed adsorption columns; providing a plurality of expandable bed column inflow and outflow control units, communicatively coupled tot said plurality of expandable bed adsorption columns, at least some of the columns connected in series; and switching at least one of said inflow and outflow control units, so as to subsequently subject said expandable bed adsorption columns to a number of process steps of a process cycle. Said switching is controlled separately so as to regulate a fluid level in the at least one expandable bed adsorption column.

In order to switch the flow control units separately, a controller can be provided. The controller can control one or more flow control units to be switched asynchronously. Also a plurality of controllers can be provided to control a plurality of flow control units independently.

According to another aspect of the invention, a chromatographic system is provided comprising: a plurality of communicatively coupled expandable bed adsorption columns, at least some of the columns connected in series; at least one of said expandable bed adsorption columns being provided with an inflow and an outflow control unit; a plurality of expandable bed column inflow and outflow control units for controlling fluid flow in said expandable bed adsorption columns; and a processor arranged to control switching of the at least one expandable bed column inflow and/or outflow control units, so as to subsequently subject said expandable bed adsorption columns to a number of process steps of a process cycle. The input and output control units are controlled separately so as to regulate a fluid level in the at least one expandable bed adsorption column.

By using expandable bed columns in combination with the method and system described herein, the process economy becomes more competitive and an expansion state of the beds can be kept relatively stable.

### DESCRIPTION

For optimal physical properties potato proteins are preferably fractionated and purified to high purity levels in a native form.

Continuous chromatography has been developed for the isolation of various compounds. This type of chromatography can be operated using a fixed bed expansion with varying flows depending on the stage of the chromatographic process of using a varying bed height in which the liquids are removed just above the bed surface using an adaptive float and/or abed height sensing device.

This latter type forms a so-called expandable filter bed (EBA) configuration, typically, wherein a flow is provided through the bed in a flow direction against the direction of gravity. This flow mode allows the filter bed to come in an unpacked state. This mode has various technical advantages using crude and particulates containing feed stream such as less fouling or clogging. The expanded mode allows higher flow rates and shorter process times, leading to higher productivities and higher efficiencies of adsorption materials.

An overview of the different continuous systems is presented by Ching and Wang (Chin C.Y. and Wang N-H. L. (2004) "Simulated Moving Bed Equipment Designs", Separation and Purification Reviews, 33, pp.77 - 155). However, various problems are met when scaling up the EBA system for large scale processing. For large scale EBA processes (e.g. agro stream such a potato fruit juice >1000 T protein year) some challenges are:
- Absolute high processing costs;
- High buffer and eluate volumes;
- Varying expansions of the column beds, which does not allow a capturing of the eluate with minimal dilution;
- Engineering limitation such as a maximum diameter and height of he columns for single columns.
- The height of the single columns taking in account the varying bed height
- Varying bed height of the column material during the various stages of the chromatography process.
- The productivity of EBA columns; many stages in a single bed EBA process lead to streams with relatively low product concentrations
- Purity of the protein isolate.
- The ability to fractionate separate proteins sufficiently
- Limited capture efficiency of a single EBA column

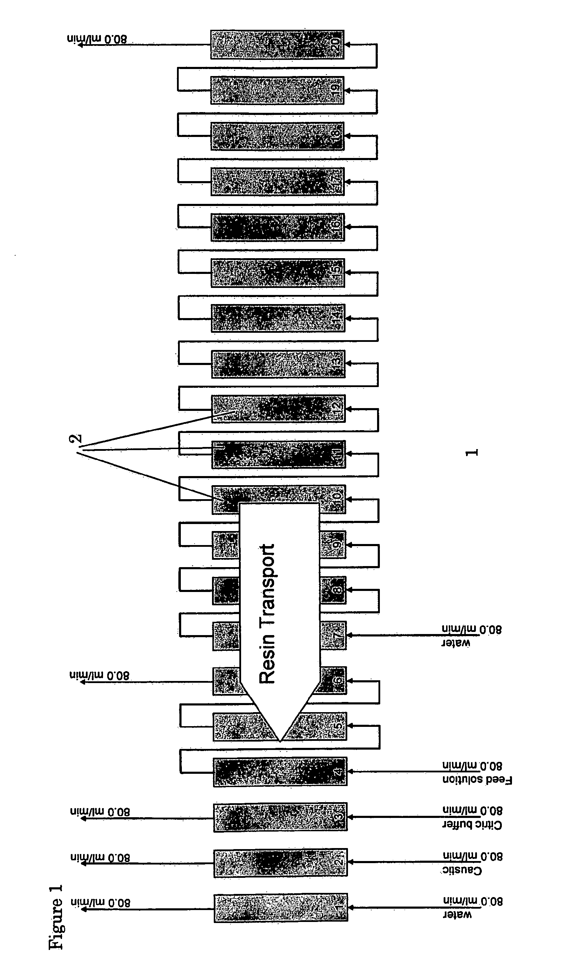

Figure 1 schematically shows a continuous multicolumn system for separating two protein fractions in a carousel type SMB unit 1. The unit 1 was formed by a Septor (SepTor Technologies B.V. The Netherlands) 20 column carousel. A pilot SepTor unit 1 for twenty expandable bed adsorption columns, (model 20-6), containing twenty columns 2 of dimensions 32 x 1000 mm, made of transparent PVC was provided at least partially connected in series. Accordingly a system is provided comprising multiple columns of an expanded bed type, that were connected to form a continuous system the columns follow a similar sequence of events during multiple cycles.

In the tests, switching was provided in a time-controlled manner; in particular, by changing the switch time of the columns at a constant flow. Accordingly the loading volume of the feed was changed to regulate a fluid level in the at least one expandable bed adsorption column. However, the carrousel type continuous chromatography EBA system has some technical drawbacks due to a rotating turntable. Another drawback of this approach is the fact that all columns switch simultaneously from one position to the next, which limits the possibility to keep the beds in a substantially constant state of expansion when the wash and elution buffer flow rates are not controlled separately.

In a series of tests, samples have been collected from the outlet streams to study the capture efficiency of the system. The presence of the two protein fractions was determined by gel electrophoresis. The concentration of total protein was determined by nitrogen analyses. To study the wash step, the conductivity was measured of the outlet of the wash column. To study the elution the UV was measured of the outlet of this step.

From these results using SDS-page electrophoresis, it was concluded that it was possible to fractionate the two proteins on this system. In addition, nitrogen analyses were used to determine to concentration of protein. It was shown that only with loading volumes considerably larger than compared to batch chromatography the efficiency starts to drop, meaning that part of the proteins breaks through. It is thus possible to load more feed with a high capture efficiency in a SMB system in which the adsorption zone consists of more columns.

Although this system may be used in other type of expandable bed continuous chromatographic separations, preferably, the system is used for providing potato protein fractions. In such a setup, the expandable bed column preferably has adsorber functionality capable of binding both fractions, which is known as a so called mixed mode adsorbent. Accordingly, preferably, the EBA columns are filled with an adsorbent that has multimodal ligands that have the ability to bind two separate components from solution.

The present system allows - contrary to batch chromatography - subsequent selective adsorption of the two components under different conditions. This can be established by - for instance - adjusting the pH between two loading zones. After the loading zone, where one of the components is captured on the resin, the effluent is directed to a second loading zone, where the second component is captured under different conditions. After loading, the columns can be washed, eluted and equilibrated before being subject to the loading again.

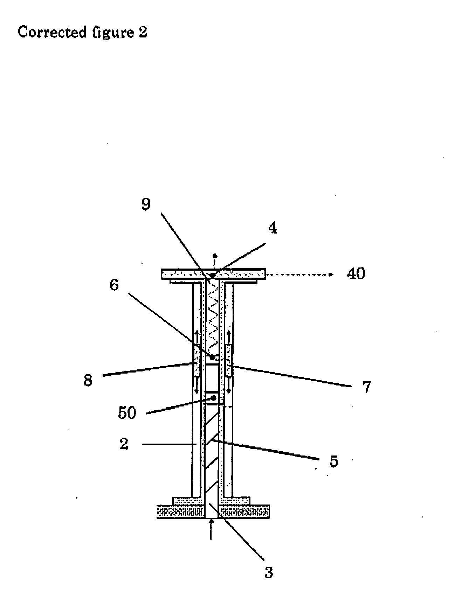

With reference to Figure 2 an embodiment is shown of an expandable bed column 2 comprising a flow controlled outflow control unit 6 in the form of a top collector 6. The collector is connected or may comprise a pump and is provided in said column 2 adapted for collecting process fluid filtered by said filter bed 5. In one process step, the process fluid is a product feed input comprising potato proteins to be isolated, in other process steps, the process fluid may be wash fluid, elute fluid, cleaning fluid, equilibration fluid, isolate fluid or waste fluid. These fluids can be collected by top collector 6, actuated by top collector actuator 7. In this embodiment, a top stratum position detector 8 is constructed and adapted to detect a top stratum position 50 of the filter bed 5 when in expanded state. In addition, a controller (not shown) is communicatively coupled to said top stratum position detector and said top collector actuator 7, for controlling movement of the top collector as a function of a detected top stratum position 50.

In particular, Figure 2 shows an expandable bed column 2 connected to a fluid inlet 3 and a fluid outlet 4 provided in top plate 40. The column 2 is dimensioned to comprise an expandable filter bed 5 between said fluid inlet 3 and said fluid outlet 4. Typically the column is about one to three meters high and about 10 tot 200 centimeters wide. The expandable filter bed 5 provides filtering for a substance that is flowing to the fluid inlet 3 and is collected by a top collector 6 held in closed vicinity of a top layer or stratum 50 filter bed 5. Since the filter bed is kept in expanded state through a balance of fluid 3 flow and gravity exerting a gravity force on the filter 5 the top stratum 50 of the filter 5 is movable in axial direction along a column axis. The optimal filter quality is obtained just near the top stratum 50 of the filter bed 5 and to this end a top collector 6 is actuated by a top collector actuator 7.

Although other embodiments may be feasible wherein the actuator 7 is internal of the column, in a preferred embodiment the top collector actuator is external of the column since this provide the most easy cleanable environment for the column 2. Although not specifically shown in Figure 2 a top stratum detector 8 is constructed to detect a top stratum 50 of the filter bed 5 when in expanded state. In this embodiment the detector 8 is integral to the actuator 7 but of course other embodiments are feasible where de top stratum position detector 8 is provided for example integral to a top collector 6 or as a separate piece.

Furthermore, separate from the detector 8 or connected thereto a controller is provided (not shown), that is communicatively coupled to the top stratum position detector 8 and the top collector actuator 7 for moving the top collector 6 as a function of a detected top stratum position 50. The control can be provided in several ways, for example the top collector can be held at a predetermined distance between a top stratum 50 of the expanded filter bed 5 and the top collector 6, but this predetermined distance can also be a predetermined range. One advantageous aspect of this embodiment is the closed environment that is offered since the collector 6 is coupled to filtered substance outlet 9 provided in the top part of the column 2 via a coiled tubing 10. The same detector 8, which in a preferred embodiment is non invasive of nature and based on optical signals, can also be used for detecting the liquid-air interface, to control the fluid flow 3. Preferably, controller 13 is arranged to maintain a distance between a top stratum 50 of an expanded filter bed 5 and said top collector 6 in a predetermined range.

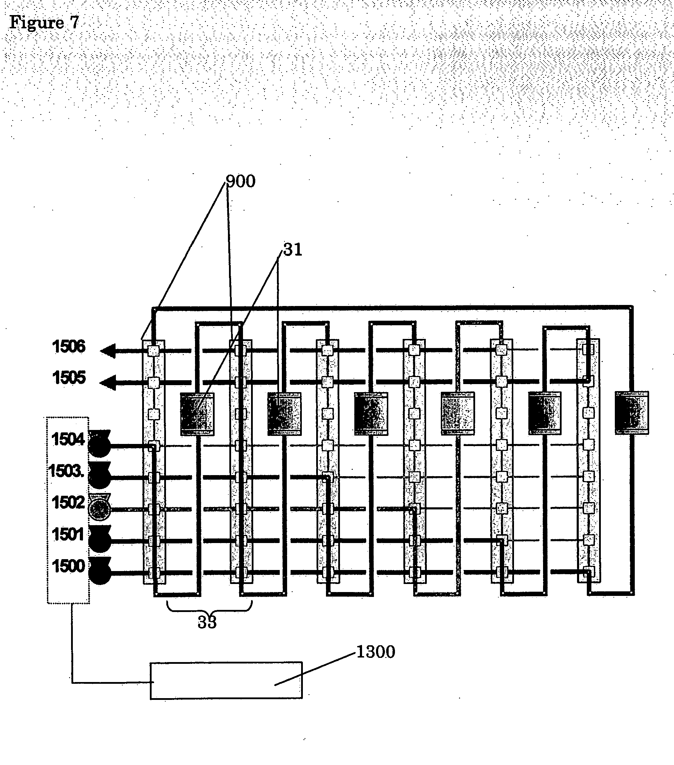

In the system configuration of Figure 2 the top stratum position detector 8 can be coupled to a processor 1300 (see Figure 7.) for regulating a fluid level of the expandable bed adsorption columns as a function a measured top stratum position 50 of said top stratum position detector 8, so as to provide an equal expansion state in said expandable bed columns.

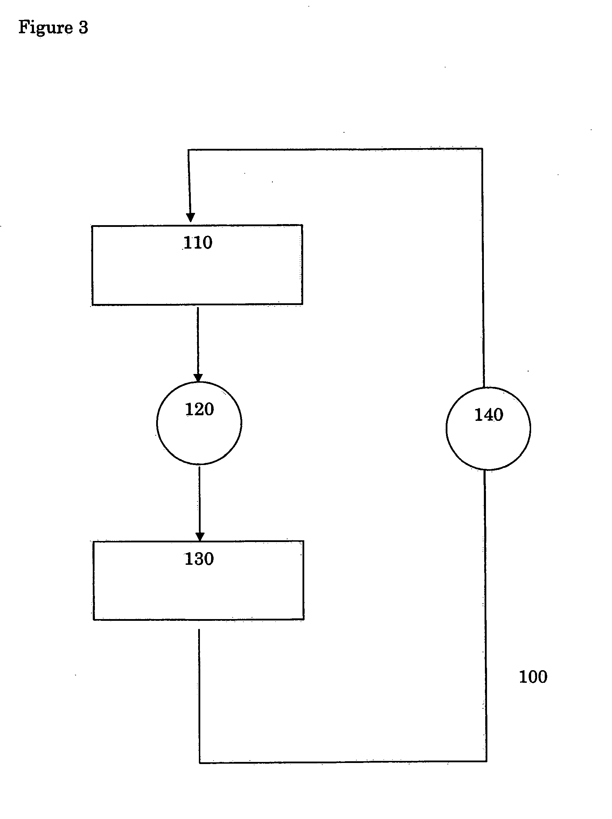

Turning to Figure 3 a first selective elution process 100 is illustrated obtaining a native potato protein isolate. The steps are performed in an iterative way to provide a continuous separation process.

Prior to the iterative process, preferably, the potato fruit juice is pre-treated. By way of example, this can be done by a divalent metal cation at a pH of 7-9, preferably 7.0-7.5, to flocculate undesired material, followed by a separation of the flocks by centrifugation. A particularly suitable divalent metal cation is Ca2+. It has been found that this pre-treatment removes undesired material such as negatively charged polymers, pectins, glycoalkaloids, and microorganisms from the potato fruit juice. In particular, the removal of pectins and glycoalkaloids is advantageous, since these compounds adhere to the potato proteins and may cause flocculation. These compounds thus may lead to an unstable protein isolate.

Subsequently, the supernatant is subjected to expanded bed adsorption chromatography as follows.

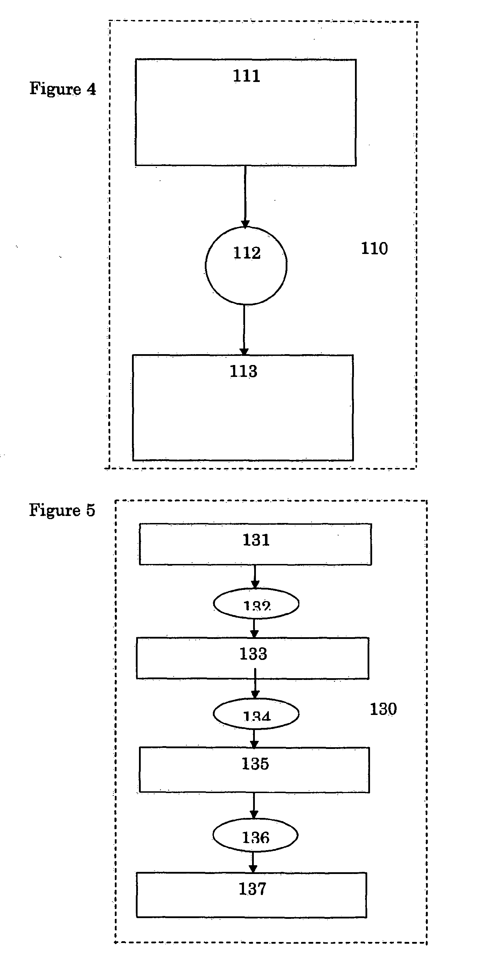

A binding action 110 includes providing a process fluid comprising potato fruit juice in at least one of the expandable bed columns 2. In this action a potato protein of interest is bound to an adsorbent provided in said at least one expandable bed column. The binding action 110 may comprise a number of pre- and post processing steps to be explained further with reference to Figure 4 and Figure 5.

After binding a switching action 120 is performed including switching at least one flow control unit. A control unit 900 (see Fig 7) comprises at least one of a valve and a pump, preferably, each control unit comprises a separate pump unit for controlling said inflow and/or outflow separately. Accordingly, for an expandable bed column an elution action 130 is initiated. Typically, for potato fruit juice, two classes of proteins of interest may be desorbed sequentially, that is, the patatin class and the protease inhibitor class referenced here above. Also, a number or even all the bound proteins may be desorbed at one time to obtain a blend of the above classes.

Column materials that bind native potato proteins include mixed-mode adsorbentia such as Amersham Streamline^{™} Direct CST I (GE Healthcare), Fastline adsorbentia (Upfront Chromatography A/S), macroporous adsorbentia such as Amberlite^{™} XAD7HP (Röhm & Haas Company) and ion exchange adsorbents (for patatin isolates and purification see G. Koningsveld, "Physico-chemical and functional properties of potato proteins", PhD thesis, Wageningen University, Wageningen, The Netherlands, 2001; for protease inhibitor isolates see L. Pouvreau, "Occurrence and physico-chemical properties of protease inhibitors from potato tuber (Solanum tuberosum)", PhD thesis, Wageningen University, Wageningen, The Netherlands, 2004). The adsorbent with adsorbed native potato proteins is subsequently eluted with a suitable eluent in order to retrieve the native potato protein isolate. The eluent preferably has a pH in the range of 4-12, more preferably in the range of 5.5-9.0.

Preferably, the native potato protein isolate has an isoelectric point above 4.8, a molecular weight of more than 5 kDa.

In a preferred embodiment using mixed-mode adsorbentia the proteins can be fractionated to both isoelectric point and molecular weight. This allows to separate the patatin and protease inhibitor fractions. Patatin isolates are eluted at a pH of 5.7-8.7, preferably at a pH of 5.8-6.2. Protease inhibitors are eluted at a pH of 5.8-12.0, preferably at a pH of 6.0-9.5.

After the elution action 130 a second switching action including switching at least one flow control unit repeats the binding action 110 for a new process fluid.

Action 130 also can comprise a number of pre- and post processing steps; it at least comprises a step of providing an elution fluid flow to elute a product of interest, typically one or of two of the patatin and protease inhibitors from at least one expandable bed column 2. Thus, during elution 130, the product of interest is eluted from the at least one expandable bed column.

To control the switching actions 120, 140 of the valves, a processor 1300 is arranged (see Figure 7). This processor 1300 essentially controls the fluid flows and directions thereof, by timely actuating a plurality of valves and/or pumps 900.

After elution action 130 a second switching action 140 is performed including switching at least one flow control unit to repeat said binding action 110 in an iterative way, until a predetermined stopping criterion is reached, which may be a time period or a concentration criterion of a specific product or fluid used during the product yielding process 100.

In a preferred mode, Figure 4 shows that the binding action 110 can also comprise a number of pre- and post processing steps, i.e. it may include a number of associated actions, in particular a regenerating action 111 as a preprocessing action, that is, an action executed prior to providing the process fluid to the expandable bed column for binding a protein component of interest including providing at least one regenerating fluid in said at least one expandable bed column 2. After the regeneration action 111, a switching action 112 is performed including switching at least one flow control unit to initiate providing of said potato fruit juice process fluid for binding the proteins of interest in step 113.

Typically, the regenerating fluid has functionality to clean, sanitize and/or strip and/or equilibrate the adsorbent prior to binding the component of interest, so that the adsorbent does not wear out in the iterative process, but can be used repeatedly as long as possible.

Also, with reference to Figure 5, the elution action 130 may further comprise a number of pre- and postprocessing steps, in particular, preferably includes a washing actions 131 and 135. The washing action 131 and 135 typically include providing a buffer in said expandable bed column to replace the process fluid in the expandable bed column 2 by said buffer fluid. After washing action 131, 135 respectively, switching actions 132 and 136 are performed. By the switching actions 132, 136 respectively, a flow control unit initiates providing elution fluid flow in steps 133 and 137; wherein subsequently, the two components of interest are eluted in a selective way as described here above; typically, by varying a pH of the elution fluid.

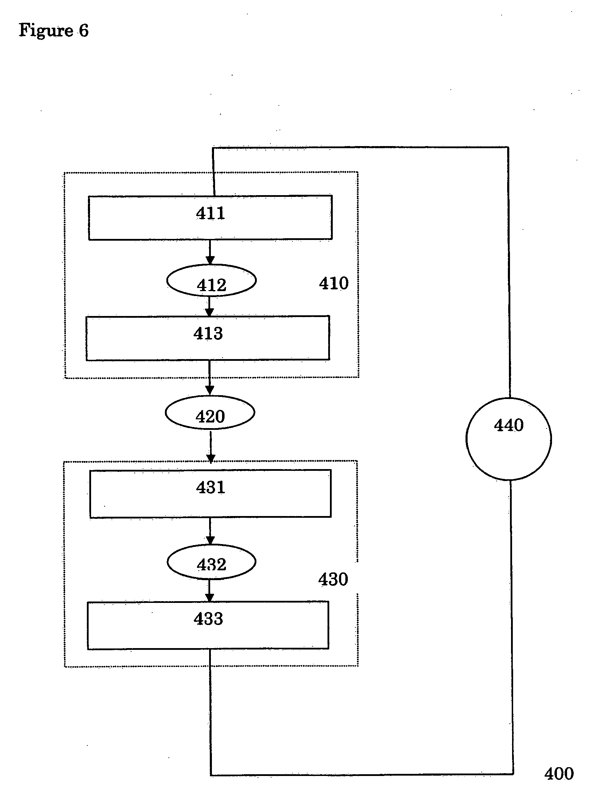

Figure 6 shows an alternative embodiment of the example process flow depicted in Figure 1. In this process flow 400, iteratively, a separation process is performed to obtain a purified potato protein isolate of interest by selective binding. Accordingly, in this embodiment, adsorber functionality is provided capable of selectively binding patatin and protease inhibitor proteins. Typically, the binding and elution actions described in Figure 3 are repeated in steps 410 and 430 with different binding conditions, to specifically bind a protein of interest. In the first step 410 a binding and elution process is performed at an elevated pH. Hereafter the effluent is adjusted to a lower pH so that a second desired potato protein fraction can bind.

Although this selective binding mode leads to lower productivity and higher buffer use compared to selective elution mode; this mode has as an advantage that the obtainable purity levels are higher. Typically, the process comprises a first binding 410 action including providing (step 411) a process fluid comprising potato fruit juice in at least one of the expandable bed columns 2 at an elevated pH. In this action a potato protein of interest is bound to an adsorbent provided in said at least one expandable bed column 2. This binding action 411 may comprise a number of pre- and post processing steps. After binding 411, a switch 412 is performed to initiate a first elution process 413, to elute a first component of interest.

Additionally, a switching action 420 is performed to initiate a second binding elution process 430 at a lower pH adjusted to a pH of 4.5-5.0, preferably to a pH of 4.8 to bind the patatin fraction in step 431. Then, a flow control unit is switched in step 432, and a second elution step 433 is commenced. This process can be repeated continuously.

### EXAMPLES

In a method for providing products involving a Bind & Elute chromatographic separation, the system can be used as shown in Figure 7. The system has Wash input flow control unit 1500, Elute input flow control unit 1501, Clean input flow control unit 1502, Equilibration input flow control unit 1503, and Product Feed control unit 1504. Furthermore, Output flows Product 1505 and Waste 1506 are provided.

The process fluids provided to said columns are switched using valves and pumps 900, thus constituting separately controlled inflow and outflow control units of the expandable bed columns 2. The inflow and outflow control units 900 are thus arranged to overcome problems with hydrodynamic bed stabilization every time a column is switched from one inlet position to the other. Specifically, the process is preferably operated with a more or less constant bed expansion throughout the entire process. In addition, or alternatively, the in- and outlets of the columns are controlled by individual (membrane) valves.

Such system allows:
· Countercurrent loading by having at least two columns connected in series in the loading zone
· Independent control of flow rates and switch times
· Continuous operation with constant bed expansions
· The top distributors of the columns can be controlled to minimize the influence of variations in the head space above the expanded beds

In this example, the assembly comprises six expandable bed adsorption columns 31. Each column is provided with an input and output flow control unit 900. An output control unit 900 of one column can form an input control unit for a next serially coupled column 2. In this scheme, a first zone 33 is formed by two serially interconnected expandable bed adsorption columns 31. The inlet of the first expandable bed column is connected to a feed pump supplying feed solution to the system. The outlet of the second expandable bed column in this series is directed to a waste effluent through one valve manifold 1. The second, third, fourth and fifth zone only comprise one single expandable bed column 31.

The expandable bed column in the second zone is connected to a pump providing an equilibration to the system. The effluent of the expandable bed column is directed to the waste outlet. The expandable bed column in the third zone is connected to a pump providing a cleaning solution or regenerant to the system. The effluent of the expandable bed column is directed to the waste outlet. The expandable bed column in the fourth zone is connected to a pump providing an elution buffer to the system. The effluent of the expandable bed column is directed to the product outlet. The expandable bed column in the fifth zone is connected to a pump providing a wash solution to the system. The effluent of the expandable bed column is directed to the product outlet.

By going through the subsequent steps, each expandable bed adsorption column is subjected to the series of steps involved in a Bind & Elute chromatography for obtaining a specific protein of interest. The loading is performed in two serially connected expandable bed adsorption columns, thereby allowing the first column to be essentially overloaded. Any product that breaks through will be captured by the next expandable bed column. Once the first expandable bed column is saturated, it will be subjected to a wash step (step b), followed by the elution step to collect the product (step c). Before being redirected towards the loading step, the expandable bed column is subjected to a cleaning process (step d) and equilibration process (step e). The expandable bed column is then ready to be used as the last cartridge in the loading zone (step f).

In one aspect of the invention, the valves can switch asynchronously, thereby controlling the residence time of the expandable bed column in each zone individually.

In principle, any zone could comprise more than one expandable bed column connected in series by adding additional valves 1 and expandable bed adsorption columns 31.

The processor 1300 is arranged to control switching of the valves in manifold 1. The switch times are preferably controlled such that the effective column transport rate is balanced with the incoming flux of components to be adsorbed.
By controlling the switch times of the two loading zones separately, the capacity utilization can be fully optimized for both components separately.
Such system uses preferably four to six columns. The use of six columns allows each of the loading steps to comprise two columns and hence a loading process that moves against a direction of the flow, also known as countercurrent process. Accordingly, the resin capacity to its full extent and hence an economically attractive process can be obtained.

In a further example, a feed composition and resin capacity is used as summarized in Table 1 based on performances in a single batch EBA column process.

**Table 1.**

| Protein | Feed concentration g/l | Capacity g/l |
|---|---|---|
| Protein fraction A | 5 | 34 |
| Protein fraction B | 6 | 43 |
| Total protein | 11 | 60 |

The process involves the adsorption and elution of two different proteins. There are two possibilities for the fractionation of the two proteins. In case of selective elution both proteins will be adsorbed and fractionated during the elution. Instead of fractionating the two protein fractions in the elution, one can also exploit the fact that optimum binding conditions for the two proteins seems to be different. The binding of protein fraction B is not very dependent on pH. The binding of protein fraction A, on the other hand, binds at pH around 5 preferably at pH4.8. This phenomenon can be exploited by implementing a two-stage adsorption with intermediate elution. The capacity of protein A and protein B is used in the calculations for selective adsorption while the capacity for the total protein is used in the calculation for selective elution.

For a static type multicolumn process the process cycle for selective adsorption is summarized in table 2.

**Table 2:**

| **Step** | **Volume** |
|---|---|
| Loading | 6.21 BV |
| Intermediate washing | 1.00 BV |
| Elution of protein fraction A | 2.00 BV |
| Equilibration (after elution A) | 1.00 BV |
| Elution of protein fraction B | 2.00 BV |
| Equilibration (after elution B) | 1.50 BV |

For a 50 cm settled bed height, in this example, the key figures of the process are:
- Column dimensions:

| | | | |
|---|---|---|---|
| o | Column diameter (ID): | 125 cm | |
| o | Settled bed height: | 50 cm | |
| o | Resin volume: | 0.61 m³ | (per column) |
| o | Resin volume: | 3.07 m³ | (total five columns) |
| o | Cycle time: | 0:26 hr | |

Based on the process cycle given in table 2, the consumption of buffers can be calculated:
- Nominal buffer consumptions:

| | | | | |
|---|---|---|---|---|
| o | Elution buffer A: | 7.09 | m³/hr 33.7 | liter/kg |
| o | Elution buffer B: | 7.09 | m³/hr 33.7 | liter/kg |
| o | Equilibration buffer (B): | 5.31 | m³/hr 25.3 | liter/kg |
| o | Equilibration buffer (A): | 3.54 | m³/hr 16.8 | liter/kg |
| o | Wash solution: | 10.6 | m³/hr 16.8 | liter/kg |

For a static type multicolumn process the process cycle for selective elution is summarized in table 3.

**Table 3**

| **Step** | **Volume** |
|---|---|
| Equilibration | 1.50 BV |
| Loading | 4.7 BV |
| Wash | 0.7 BV |
| Elution of protein fraction P1 | 4.00 BV |
| Elution of protein fraction P2 | 2.00 BV |

Since the adsorption zone will contain more columns, the length of each column can be decreased to 40 cm. The key figures of the process are:
- Column dimensions:

| | | | |
|---|---|---|---|
| o | Column diameter (ID): | 125 cm | |
| o | Settled bed height: | 40 cm | |
| o | Resin volume: | 0.49 m³ | (per column) |
| o | Resin volume: | 2.96 m³ | (total six columns) |
| o | Cycle time: | 0:17 hr | |

Based on the process cycle given in table 2, the consumption of buffers can be calculated:
- Nominal buffer consumptions:

| | | | |
|---|---|---|---|
| o | Wash buffer | 2.1 m³/hr | 16.1 |
| o | Elution buffer P1: | 11.8 m³/hr | 88.5 liter/kg |
| o | Elution buffer P2: | 5.9 m³/hr | 44.2 liter/kg |
| o | Equilibration buffer: | 4.4 m³/hr | 33.2 liter/kg |

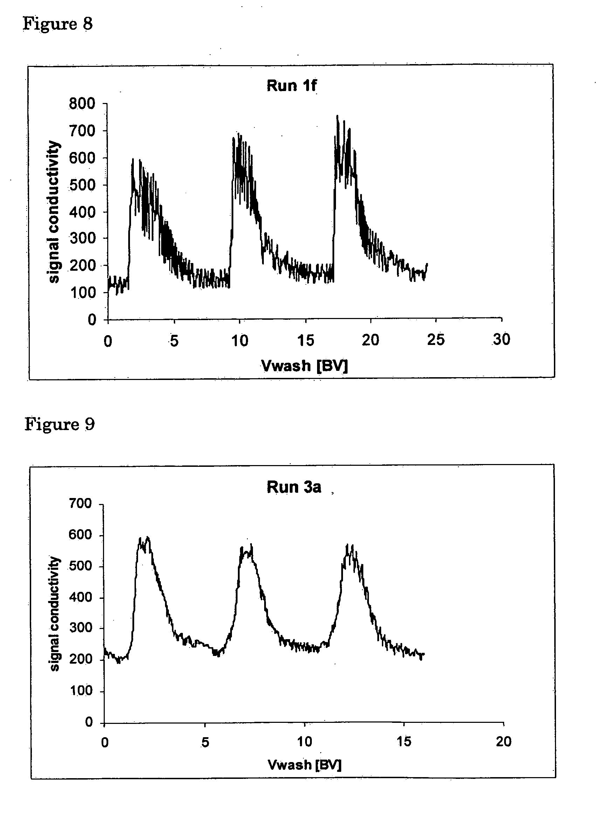

Figure 8 and Figure 9 shows conductivity profiles of wash effluent for differing head volumes using a 20 column pilot carousel unit as depicted in figure 1. The dimensions of the columns 2 used 32 x 1000 mm. In Figure 8 the head space was kept at was approximately 25 cm; in Figure 9 the head space was approximately 15 cm. Comparison of the figures, show that the wash efficiency is depending on the bed expansion. In some experiments the expanded bed height was more than 25 cm. In these cases the necessary wash volume to wash the column was approximately 5 BV. Here the term BV indicates "Buffer Volume which is defined as the filter bed volume in unexpanded state. When the head space was decreased to about 15 cm the wash volume needed decreased to approximately 3 BV. The same kind of results was found for elution.

Accordingly, in a preferred embodiment, the system 1 of Figure 1 comprises inflow and/or outflow control units control programmed to control a head volume in said expandable bed column as a function of fluid inflow velocity, fluid viscosity and fluid outflow velocity; preferably, while keeping a constant fluid outflow velocity. Preferably, the head volume height is kept below 20 cm, more preferably even as below 15 cm or even below 10 cm.

From this it can be concluded that the volume of clarified liquid above the expanded bed should be controlled within certain margins. In case the liquid level above the bed is too close to the top of the expanded bed, carry-over of chromatography beads cannot be avoided. In case the liquid level above the expanded bed is too high, the back mixing in the liquid above the bed will lead to reduced efficiency of the chromatography process. This will result in undesired high buffer consumptions for - for instance - washing and equilibration of the bed and in a dilute eluted product.

The complexities of combining expanded bed chromatography in a continuous multicolumn system are:
Variations in the linear flow rate in each of the columns should be kept within certain margins in order to (a) avoid settling of the bed in the bottom of the column at too low linear flow rates and (b) avoid carry-over of smaller particles from the top of the bed. Variations in the flow rates occur since the flow rates in the different steps of the continuous chromatography process differ;

Accordingly, the following covers some aspects of the invention:
The flow rate of the influent and effluent of each individual expanded bed column in the system needs to be carefully adjusted to each other. One of the additional complexities is that the effluent of one column can be the influent of a next column in case two columns are connected in series. Preferably, the adjustment of each of the effluent pumps is automatically controlled by monitoring the levels of the expanded bed and the liquid above the expanded bed in the each of the columns;
· The switching time of the individual columns are preferably not identical and should be carefully adjusted to each other. This requires asynchronous switching schemes that are not only controlled by time but also by the flow rates in each zone;
In one embodiment, the valves that control the influent and effluent of each column do not switch simultaneously. Once a column is subjected to the next step in the chromatography process, the fluid inside the column will be gradually displaced by the new incoming solution. Only once all or a significant portion of the interstitial liquid is displaced, the effluent valve should switch. Since the level of the expanded bed - and hence the liquid level - in the columns vary across the cycle of events in the process, the liquid volume in the column will also vary. The switching of the valves is preferably controlled such that this is taken into account.

Although the invention has been described with reference to the exemplary embodiments, the invention is not limited thereto. Indeed, for example, although the examples show a flow controlled manner of adjusting the regulating a fluid level in the at least one expandable bed adsorption columns; careful process design can also provide a time-controlled manner, since the expansion behavior of the filter bed is known once the feed flows and there characteristics, in particular, viscosities are known or measured, and are controlled carefully. In addition, the valves are preferably organized in a modular fashion to allow minimization of the critical dead volumes in the process.

In addition, the use of sanitary membrane valves may also allow cleaning of the system to (bio-) pharmaceutical standards. Indeed, batch wise Expanded Bed Adsorption has been tested and is being used for the recovery of monoclonal antibodies from unclarified cell culture broth. In most cases, the EBA recovery process relies on the use of Protein A chromatography resins. Considering the fact that the scale of monoclonal antibody production is increasing rapidly, the use of batch-wise EBA will soon reach its limitations. The advantages of using EBA for directly recovering monoclonal antibodies from the unclarified broth will then no longer compensate the disadvantages of water consumption and reduced resin capacity. The use of multicolumn chromatography will help to overcome these disadvantages for this type of applications too. The use of EBA in multiple connected columns is not limited to monoclonal antibodies, but can also be employed for other recombinant proteins or biopharmaceutical products, including plasmid DNA, from unclarified fermentation broths or cell culture broths.

These and other modifications are deemed to fall within the scope of the invention, as claimed in the annexed claims.

## Claims

1. A chromatographic separation method comprising:
- providing a plurality of expandable bed adsorption columns;
- providing a plurality of expandable bed column inflow and outflow control units, communicatively coupled to said plurality of expandable bed adsorption columns, at least some of the columns connected in series to form a zone for carrying out one of a loading, washing, elution, cleaning and equilibration step; and
- switching at least one of said inflow and outflow control units, so as to subsequently subject said expandable bed adsorption columns to a number of process steps of a process cycle; wherein said control units each have a pump and a valve to adjust the flow rate of each individual column relative to each other and to adjust a switching time of the individual column relative to each other, wherein the switching time is adjusted as a function of process time and flow rates in each zone in an asynchronous switching scheme to regulate an expanded bed level in each of the expandable bed adsorption columns to keep the beds in an expanded state.

2. A method according to claim 1, wherein said expandable bed column has adsorbed functionality capable of binding protein from agro and/or dairy sources.

3. Method according to claim 2 wherein a native potato protein isolate comprising patatin and/or protease inhibitor are separated from a potato fruit juice.

4. Method according to claim 3, wherein said patatin isolate having an isoelectric point of below 5.8, preferably of 4.8 - 5.5, a molecular weight of more than 30 kDa, preferably more than 35 kDa.

5. Method according to claim 3, wherein said protease inhibitor isolate has an isoelectric point above 5.5, preferably above 5.8, a molecular weight of below 35 kDa, preferably of 5-30 kDa.

6. Method according to claim 1 wherein biopharmaceutical recombinant proteins are separated from fermentation broths or cell culture broths.

7. A method according to claim 2, wherein said inflow and/or outflow control units control a head volume in said expendable bed column as a function of fluid inflow velocity, fluid viscosity and fluid outflow velocity.

8. A method according to claim 7, wherein, said head volume is controlled while keeping a constant fluid outflow velocity.

9. A method according to claim 7, further comprising detecting a top stratum position of a bed in expanded state; and moving a top collector as a function of a detected top stratum position for collecting the fluid outflow.

10. A method according to claim 9, further comprising maintaining a distance between a top stratum of an expanded filter bed and said top collector in a predetermined range.

11. A method according to claim 2, wherein said switching is provided in a time controlled manner.

12. A method according to claim 1, wherein said process steps include, for obtaining a protein isolate of an agro or dairy source stream, in an iterative way:
- a binding action including providing a process fluid comprising potato fruit juice in at least one of said plurality of expandable bed columns for binding a protein of interest to an adsorbent provided in said at least one expandable bed column; and
- an eluting action including providing a elution fluid flow in said at least one expandable bed column to elute the protein of interest from the at least one expandable bed column; wherein
- a first switching action including switching at least one flow control unit initiates the elution action; and wherein
- a second switching action including switching at least one flow control unit initiates said binding action.

13. A method according to claim 12, wherein said expendable bed column has adsorber functionality capable of binding two or more protein fractions; and wherein said elution action further includes:
- a third switching action including switching at least one flow control unit to initiate providing a second elution fluid flow to the expandable bed column; and
- providing said second elution fluid flow in said expandable bed column to selectively elute one of said protein fractions.

14. A method according to claim 12, wherein said expandable bed column has adsorbed functionality capable of selectively binding two or more protein fractions; and wherein said binding action further includes:
- a fourth switching action including switching at least one flow control unit to initiate providing a second process fluid to the expandable bed column; and
- providing said second process fluid in said expandable bed column to selectively bind one of said protein fractions.

15. A method according to claim 12, wherein said binding action further includes;
- a regenerating action including providing at least one regenerating fluid in said at least one expandable bed column; followed by
- a fifth switching action including switching at least one flow control unit to initiate said providing of said process fluid for binding said protein of interest.

16. A method according to claim 15 wherein said at least one regenerating fluid has functionality to clean, sanitize and/or strip and/or equilibrate the adsorbers prior to binding the component of interest.

17. A method according to claim 12, wherein said elution action further includes
- a washing action including providing a buffer in said expandable bed column to displace said process fluid from a dead volume in said at least one expandable bed column by said buffer; followed by
- a sixth switching action including switching at least one flow control unit to initiate providing said elution fluid flow to elute the protein of interest.

18. A chromatographic system comprising:
- a plurality of communicatively coupled expendable bed adsorption columns, at least some of the columns connected in series to define a zone for carrying out one of a loading, washing, elution, cleaning and equilibration step; wherein said expandable bed adsorption columns each being provided with an inflow and an outflow control unit having a pump and a valve for controlling fluid flow in said expandable bed adsorption columns; and wherein
- a processor is arranged to control switching of the control units to adjust the flow rate of each individual column relative to each other and to adjust a switching time of the individual colunms relative to each other, wherein the switching time is adjusted as a function of process time and flow rates in each zone in an asynchronous switching scheme, so as to subsequently subject said expandable bed adsorption columns to said loading, washing, elution, cleaning and equilibration steps of a process cycle, wherein said input and output control units are controlled separately so as to regulate a expanded bed level in each of the expandable bed adsorption columns.

19. A chromatographic system according to claim 18, said outflow control unit comprising:
- a top collector provided in said column adapted for collecting substance filtered by said filter bed;
- a top collector actuator;
- a top stratum position detector constructed and adapted to detect a top stratum position of the filter bed when in expanded state; and
- a controller communicatively coupled to said top stratum position detector and said top collector actuator, for moving the top collector as a function of a detected top stratum position.

20. A system according to claim 19, wherein said controller is arranged to maintain a distance between a top stratum of an expanded filter bed and said top collector in a predetermined range.

21. A system according to claim 19, wherein a top stratum position detector of one expandable bed adsorption column is coupled to said processor for regulating a fluid level of at least another expandable bed adsorption column as a function a measured top stratum position of said top stratum position detector, so as to provide an equal expansion state in said expandable bed columns.

22. A system according to claim 19, wherein said number of columns ranges between 4 and 20 and wherein said column height and diameter are smaller than 2.5 meter.

## Patentansprüche

1. Chromatographisches Separationsverfahren mit:
- Vorsehen einer Mehrzahl von expandierbaren Bett-Adsorptionssäulen;
- Vorsehen einer Mehrzahl von expandierbaren Bett-Säuleneinlass- und -auslasssteuereinheiten, die kommunizierend mit der Mehrzahl von expandierbaren Bett-Adsorptionssäulen gekoppelt sind, wobei mindestens einige der Säulen in Reihe verbunden sind, um eine Zone auszubilden, um einen von einem Beladungsschritt, einem Waschschritt, einem Eluierschritt, einem Reinigungsschritt und einem Ausgleichsschritt durchzuführen; und
- Schalten von mindestens einer der Einlass- und Auslasssteuereinheiten, damit in den expanierbaren Bett-Adsorptionssäulen nacheinander eine Anzahl von Prozessschritten eines Prozesszyklus durchgeführt wird; wobei die Steuereinheiten jeweils eine Pumpe und ein Ventil aufweisen, um die Strömungsrate von jeder individuellen Säule relativ zueinander einzustellen und um eine Schaltzeit der individuellen Säule relativ zueinander einzustellen, wobei die Schaltzeit als eine Funktion von Prozesszeit und Strömungsraten in jeder Zone in einem asynchronen Schaltschema eingestellt wird, um einen expandierten Bett-Pegel in jeder der expandierbaren Bett-Adsorptionssäulen zu regulieren, um die Betten in einem expandierten Zustand zu halten.

2. Verfahren nach Anspruch 1, bei dem die expandierbare Bett-Säule eine Adsorberfunktionalität hat, um Protein von Landwirtschafts- und/oder Milchproduktquellen zu binden.

3. Verfahren nach Anspruch 2, bei dem ein natives Kartoffelproteinisolat, das Patatin- und/oder Proteasehemmer enthält, von einem Kartoffelfruchtsaft separiert wird.

4. Verfahren nach Anspruch 3, bei dem das Patatinisolat einen isoelektrischen Punkt unter 5,8, vorzugsweise 4,8 - 5,5, und ein Molekulargewicht von mehr als 30 kDa, vorzugsweise mehr als 35 kDa, hat.

5. Verfahren nach Anspruch 3, bei dem das Proteasehemmerisolat einen isoelektrischen Punkt über 5,5, vorzugsweise über 5,8, und ein Molekulargewicht von weniger 35 kDa, vorzugsweise von 5-30 kDa, hat.

6. Verfahren nach Anspruch 1, bei dem biopharmazeutische rekombinante Proteine von Fermentationsbrühen oder Zellkulturbrühen separiert werden.

7. Verfahren nach Anspruch 2, bei dem die Einlass- und/oder Auslasssteuereinheiten ein Kopfvolumen in der expandierbaren Bett-Säule als eine Funktion der Fluideinlassgeschwindigkeit, der Fluidviskosität und der Fluidauslassgeschwindigkeit steuern.

8. Verfahren nach Anspruch 7, bei dem das Kopfvolumen gesteuert wird, während eine konstante Fluidauslassgeschwindigkeit beibehalten wird.

9. Verfahren nach Anspruch 7, außerdem mit: Erfassen einer Position einer oberen Schicht von einem Bett im expandierten Zustand; und Bewegen eines oberen Kollektors als eine Funktion der Position der oberen Schicht zum Sammeln des Fluidauslasses.

10. Verfahren nach Anspruch 9, außerdem mit: Beibehalten einer Distanz zwischen einer oberen Schicht eines expandierten Filterbetts und dem oberen Kollektor innerhalb eines vorbestimmten Bereichs.

11. Verfahren nach Anspruch 2, bei dem das Schalten in einer zeitgesteuerten Weise durchgeführt wird.

12. Verfahren nach Anspruch 1, bei dem die Prozessschritte, um ein Proteinisolat von einer Landwirtschafts- oder Milchproduktquellenströmung in einer iterativen Weise zu erhalten, umfassen:
- einen Bindungsvorgang, der das Vorsehen eines Prozessfluids, das Kartoffelfruchtsaft enthält, in mindestens einer von der Mehrzahl von expandierbaren Bett-Säulen beinhaltet, um ein Protein von Interesse an ein Adsorptionsmittel zu binden, das in der mindestens einen expandierbaren Bett-Säule vorgesehen ist; und
- einen Eluiervorgang, der das Vorsehen einer Eluierfluidströmung in der mindestens einen expandierbaren Bett-Säule beinhaltet, um das Protein von Interesse von der mindestens einen expandierbaren Bett-Säule zu eluieren; wobei
- ein erster Schaltvorgang, der das Schalten von mindestens einer Strömungssteuereinheit beinhaltet, den Eluiervorgang einleitet; und wobei
- ein zweiter Schaltvorgang, der das Schalten von mindestens einer Strömungssteuereinheit beinhaltet, den Bindungsvorgang einleitet.

13. Verfahren nach Anspruch 12, bei dem die expandierbare Bett-Säule eine Adsorberfunktionalität hat, um zwei oder mehr Proteinfraktionen zu binden; und bei dem der Eluiervorgang außerdem umfasst:
- einen dritten Schaltvorgang, der das Schalten von mindestens einer Strömungssteuereinheit beinhaltet, um das Vorsehen einer zweite Eluierfluidströmung zu der expandierbaren Bett-Säule einzuleiten; und
- das Vorsehen der zweiten Eluierfluidströmung in der expandierbaren Bett-Säule, um selektiv eine der Proteinfraktionen zu eluieren.

14. Verfahren nach Anspruch 12, bei dem die expandierbare Bett-Säule eine Adsorberfunktionalität hat, um zwei oder mehr Proteinfraktionen selektiv zu binden; und bei dem der Bindungsvorgang außerdem umfasst:
- einen vierten Schaltvorgang, der das Schalten von mindestens einer Strömungssteuereinheit beinhaltet, um das Vorsehen eines zweiten Prozessfluids zu der expandierbaren Bett-Säule einzuleiten; und
- das Vorsehen des zweiten Prozessfluids in der expandierbaren Bett-Säule, um selektiv eine der Proteinfraktionen zu binden.

15. Verfahren nach Anspruch 12, bei dem der Bindungsvorgang außerdem umfasst:
- einen Regenerationsvorgang, der das Vorsehen von mindestens einem Regenerationsfluid in der mindestens einen expandierbaren Bett-Säule beinhaltet, gefolgt von
- einem fünften Schaltvorgang, der das Schalten von mindestens einer Strömungssteuereinheit beinhaltet, um das Vorsehen des Prozessfluids zum Binden des Proteins von Interesse einzuleiten.

16. Verfahren nach Anspruch 15, bei dem das mindestens eine Regenerationsfluid die Funktionalität hat, die Adsorptionsmittel zu reinigen, keimfrei zu machen und/oder abzustreifen und/oder auszugleichen, und zwar vor der Bindung der Komponente von Interesse.

17. Verfahren nach Anspruch 12, bei dem der Eluiervorgang außerdem umfasst:
- einen Waschvorgang, der das Vorsehen eines Puffers in der expandierbaren Bett-Säule beinhaltet, um das Prozessfluid aus einem toten Volumen in der mindestens einen expandierbaren Bett-Säule durch den Puffer zu verdrängen; gefolgt von
- einem sechsten Schaltvorgang, der das Schalten von mindestens einer Strömungssteuereinheit beinhaltet, um das Vorsehen der Eluierfluidströmung einzuleiten, um das Protein von Interesse zu eluieren.

18. Chromatographisches System mit:
- einer Mehrzahl von kommunizierend gekoppelten expandierbaren Bett-Adsorptionssäulen, wobei mindestens einige der Säulen in Reihe verbunden sind, um eine Zone auszubilden, um einen von einem Beladungsschritt, einem Waschschritt, einem Eluierschritt, einem Reinigungsschritt und einem Ausgleichsschritt durchzuführen; wobei jede der expandierbaren Bett-Adsorptionssäulen mit einer Einlass- und einer Auslasssteuereinheit versehen ist, die eine Pumpe und ein Ventil haben, um die Fluidströmung in den expandierbaren Bett-Adsorptionssäulen zu steuern; und wobei
- ein Prozessor ausgestaltet ist, um das Schalten der Steuereinheiten zu steuern, um die Strömungsrate jeder individuellen Säule relativ zueinander einzustellen und um eine Schaltzeit der individuellen Säulen relativ zueinander einzustellen, wobei die Schaltzeit als eine Funktion von Prozesszeit und Strömungsraten in jeder Zone in einem asynchronen Schaltschema eingestellt wird, um so nacheinander in den expandierbaren Bett-Adsorptionssäulen die Beladungs-, Wasch-, Eluier-, Reinigungs- und Ausgleichsschritte eines Prozesszyklus durchzuführen, wobei die Einlass- und Auslasssteuereinheiten separat gesteuert werden, um einen expandierten Bett-Pegel in jeder der expandierbaren Bett-Adsorptionssäulen zu regulieren.

19. Chromatographisches System nach Anspruch 18, bei dem die Auslasssteuereinheit aufweist:
- einen oberen Kollektor, der in der Säule vorgesehen und ausgestaltet ist, um eine durch das Filterbett gefilterte Substanz zu sammeln;
- ein Betätigungsmittel für den oberen Kollektor;
- einen Positionsdetektor für die obere Schicht, der konstruiert und ausgestaltet ist, um eine obere Position der Schicht des Filterbetts zu erfassen, wenn sich dieses im expandierten Zustand befindet; und
- eine Steuerung, die kommunizierend mit dem Positionsdetektor für die obere Schicht und dem Betätigungsmittel für den oberen Kollektor gekoppelt ist, um den oberen Kollektor als eine Funktion einer erfassten Position der oberen Schicht zu bewegen.

20. System nach Anspruch 19, bei der die Steuerung ausgestaltet ist, um eine Distanz zwischen einer oberen Schicht eines expandierten Filterbetts und dem oberen Kollektor innerhalb eines vorbestimmten Bereichs zu halten.

21. System nach Anspruch 18, bei dem ein Positionsdetektor für die obere Schicht von einer expandierbaren Bett-Adsorptionssäule mit dem Prozessor gekoppelt ist, um einen Fluidpegel von mindestens einer anderen expandierbaren Bett-Absorptionssäule als eine Funktion einer gemessenen Position der oberen Schicht durch den Positionsdetektor der oberen Schicht so zu regulieren, um einen gleichen Expansionszustand in den expandierbaren Bett-Säulen zu bewirken.

22. System nach Anspruch 19, bei dem die Anzahl von Säulen von 4 bis 20 reicht, und wobei die Säulenhöhe und der Säulendurchmesser weniger als 2,5 Meter betragen.

## Revendications

1. Procécé de séparation chromatographique comprenant les étapes consistant à :
✔ prévoir une pluralité de colonnes d'adsorption sur lit expansible ;
✔ prévoir une pluralité d'unités de commande d'écoulement d'entrée et découlement de sortie de colonne sur lit expansible, couplées en communication à ladite pluralité de colonnes d'adsorption sur lit expansible, au moins certaines des colonnes étant raccordées en série afin de former une zone pour réaliser une étape parmi les étapes de chargement, de lavage, d'élution, de nettoyage et d'équilibrage ; et
✔ commuter au moins l'une desdites unités de commande d'écoulement entrant et d'écoulement sortant, afin de soumettre ensuite lesdites colonnes d'adsorption sur lit expansible à un certain nombre d'étapes d'un cycle de traitement ; dans lequel lesdites unités de commande ont chacune une pompe et une soupape pour ajusteur le débat de chaque colonne individuelle les unes par rapport aux autres et pour ajuster un temps de commutation des colonnes individuelle les unes par rapport aux autres, dans lequel le temps de commutation est ajusté en fonction du temps de traitement et des débits dans chaque zone selon un schéma de computation asynchrone afin de réguler un niveau de lit expansé dans chacune des colonnes d'adsorption sur lit expansible pour maintenir les lits dans un état expansé.

2. Procédé selon la revendication 1, dans lequel ladite colonne sur lit expansible a une fonctionnalité d'adsorbeur pouvant lier les protéines provenant des sources agroalimentaires et/ou de lait.

3. Procédé selon la revendication 2, dans lequel un isolat de protéine de pomme de terre native comprenant de la palatine et/ou un inhibiteur de protéase sont séparés d'un jus de pomme de terre.

4. Procédé selon la revendication 3, dans lequel ledit isolat de patatine a un point isoélectrique inférieur à 5,8, de préférence entre 4,8 et 5,5, un poids moléculaire supérieur à 30 kDa, de préférence supérieur à 35 kDa.

5. Procédé selon la revendication 3, dans lequel ledit isolat d'inhibiteur de protéase a un point isoélectrique supérieur à 5,5, de préférence supérieur à 5,8, un poids moléculaire inférieur à 35 kDa, de préférence de 5 à 30 kDa,

6. Procédé selon la revendication 1, dans lequel les protéines recombinées biopharmaceutiques sont séparées des bouillons de fermentation ou des bouillons de culture cellulaire.

7. Procédé selon la revendication 2, dans lequel lesdites unités de commande d'écoulement entrant et/ou d'écoulement sortant commandent un volume de tête dans ladite colonne sur lit expansible en fonction de la vitesse de l'écoulement entrant de fluide, de la viscosité de fluide et/ou de la vitesse de l'écoulement sortant de fluide.

8. Procédé selon la revendication 7, dans lequel ledit volume de tête est commandé tout en maintenant une vitesse d'écoulement sortant de fluide constante.

9. Procédé selon la revendication 7, comprenant en outre l'étape consistant à détecteur une position de couche supérieure d'un lit à l'état expansé ; et l'étape consistant à déplacer un collecteur supérieur en fonction d'une position de couche supérieure détectée pour collecter l'écoulement sortant de fluide.

10. Procédé selon la revendication 9, comprenant en outre l'étape consistant à maintenir une distance entre une couche supérieure d'un lit de filtre expansé et ledit collecter supérieur dans une plage prédéterminée.

11. Procédé selon la revendication 2, dans lequel ladite étape de commutation est prévue d'une manière contrôlée par le temps.

12. Procédé selon la revendication 1, dans lequel lesdites étapes de traitement comprennent, pour obtenir un isolat de protéine d'un flux de source agroalimentaire ou de lait, d'une manière itérative :
✔ une action de liaison comprenant l'étape consistant à prévoir un fluide de traitement comprenant un jus de pomme de terre dans au moins l'une de ladite pluralité de colonnes sur lit expansible pour lier une protéine d'intérêt à un adsorbant prévu dans ladite au moins une colonne sur lit expansible ; et
✔ une action d'élution comprenant l'étape consistant à prévoir en écoulement de fluide d'élution dans ladite au moins une colonne sur lit expansible afin d'éluer la protéine d'intérêt de la au moins une colonne sur lit expansible ; dans lequel :
✔ une première action de commutation comprenant l'étape consistant à commuter au moins une unité de commande d'écoulement initie l'action d'élution ; et dans lequel :
✔ une deuxième action de commutation comprenant l'étape consistant à commuter au moins une unité de commande d'écoulement initie ladite action de liaison.

13. Procédé selon la revendication 12, dans lequel ladite colonne sur lit expansible a une fonctionnalité d'adsorbeur pouvant relier deux fractions de protéine ou plus ; et dans lequel ladite action d'élution comprend en outre :
✔ une troisième action de commutation comprenant la commutation d'au moins une unité de commande d'écoulement pour initier l'étape consistant à prévoir un deuxième écoulement de fluide d'élution sur la colonne sur lit expansible ; et
✔ prévoir ledit deuxième écoulement de fluide d'élution dans ladite colonne sur lit expansible pour éluer sélectivement l'une desdites fractions de protéine.

14. Procédé selon la revendication 12, dans lequel ladite colonne sur lit expansible a une fonctionnalité d'adsorbeur pouvant relier sélectivement deux fractions de protéine ou plus ; et dans lequel ladite faction de liaison comprend en outre :
✔ une quatrième action de commutation comprenant la commutation d'au moins une unité de commande d'écoulement pour initier l'étape consistant à prévoir un deuxième fluide de traitement dans la colonne sur lit expansible ; et
✔ prévoir ledit deuxième fluide de traitement dans ladite colonne sur lit expansible pour relier sélectivement l'une desdites fractions de protéine.

15. Procédé selon la revendication 12, dans lequel ladite action de liaison comprend en outre :
✔ une action de régénération comprenant l'étape consistant à prévoir au moins en fluide de régénération dans ladite au moins une colonne sur lit expansible ; suivis par :
✔ une cinquième action de commutation comprenant l'étape consistant à commuter au moins une unité de commande d'écoulement pour initier ladite étape consistant à prévoir ledit fluide de traitement pour lier ladite protéine d'intérêt.

16. Procédé selon la revendication 15, dans lequel ledit au moins un fluide de régénération a la fonctionnalité de nettoyer, désinfecter et/ou retirer et/ou équilibrer les absorbeurs avant de relier le composant d'intérêt.

17. Procédé selon la revendication 12, dans lequel ladite action d'élution comprend en outre :
✔ une action de lavage comprenant l'étape consistant à prévoir une solution tampon dans ladite colonne sur lit expansible pour déplacer ledit fluide de traitement à partir d'un volume mort dans ladite une colonne sur lit expansible par ladite solution tampon ; suivie par :
✔ une sixième action de commutation comprenant l'étape consistant à commuter au moins une unité de commande d'écoulement pour initier l'étape consistant à prévoir ledit écoulement de fluide d'élution pour éluer la protéine d'intérêt.

18. Système chromatographique comprenant :
✔ une pluralité de colonnes d'adsorption sur lit expansible couplées en communication, au moins certains dans colonnes étant raccordées en série pour définir une zone afin de réaliser une étape parmi les étapes de chargement, lavage, élution, nettoyage et équilibrage ; dans lequel :
✔ lesdites colonnes d'absorption sur lit expansible étant chacune prévue avec une unité de commande d'écoulement entrant et d'écoulement sortant ayant une pompe et une soupape pour contrôler l'écoulement de fluide dans ladite colonne d'adsorption sur lit expansible ; et dans lequel :
✔ un professeur est agencé pour contrôler la commutation des unités de commande afin d'ajuster le débit de chaque colonne individuelle les unes par rapport aux autres et pour ajuster un temps de commutation des colonnes individuelles les unes par rapport aux autres, dans lequel le temps de commutation est ajusté en fonction d'un temps de traitement et des débits dans chaque zone selon un schéma de commutation asynchrone, afin de soumettre ensuite lesdites colonnes d'adsorption sur lit expansible auxdites étapes de chargement, de lavage, d'élution, de nettoyage et d'équilibrage d'un cycle de traitement, dans lequel lesdites unités de commande d'entrée et de sortie, sont commandées séparément afin de réguler un niveau de lit expansé dans chacune des colonnes d'adsorption sur lit expansible.

19. Système chromatographique selon la revendication 18, ladite unité de commande d'écoulement sortant comprenant :
✔ un collecteur supérieure prévu dans ladite colonne, adapté pour collecter la substance filtrée par ledit lit de filtre :
✔ un actionneur de collecteur supérieur ;
✔ un détecteur de position de couche supérieure construit et adapté pour détecter une position de couche supérieure du lit de filtre lorsqu'il est à l'état expansé ; et
✔ un contrôleur couplé en communication avec ledit détecteur de position de couche supérieure et ledit actionneur de collecteur supérieur, pour déplacer le collecteur supérieure en fonction d'une position de couche supérieure détectée.

20. Système selon la revendication 19, dans lequel ledit contrôleur est agencé pour maintenir une distance entre une couche supérieure d'un lit de filtre expansé et ledit collecteur supérieur dans une plage prédéterminée.

21. Système selon la revendication 19, dans lequel un détecteur de position de couche supérieure d'une colonne d'adsorption sur lit expansible est couplé audit professeur pour réguler un niveau de fluide d'au moins une autre colonne d'adsorption sur lit expansible on fonction, d'une position de couche supérieure mesurée dudit détecteur de position de couche supérieure, afin de fournir un état d'expansion identique dans lesdites colonnes sur lit expansible.

22. Procédé selon la revendication 19, dans lequel ledit nombre de colonnes est compris entre 4 et 20, et dans lequel ladite hauteur et ledit diamètre de colonne sont inférieurs à 2,5 mètres.
